## Europäisches Patentamt
## European Patent Office
## Office européen des brevets

(19)

(11) Veröffentlichungsnummer: **0 080 179**
**B1**

(12) **EUROPÄISCHE PATENTSCHRIFT**

(45) Veröffentlichungstag der Patentschrift:
29.05.85

(21) Anmeldenummer: 82110683.8

(22) Anmeldetag: 19.11.82

(51) Int. Cl.⁴: **A 61 M 1/00, A 61 M 27/00**

(54) Vorrichtung für eine medizinische Saugdrainage.

(30) Priorität: 21.11.81 DE 3146266

(43) Veröffentlichungstag der Anmeldung:
01.06.83 Patentblatt 83/22

(45) Bekanntmachung des Hinweises auf die Patenterteilung:
29.05.85 Patentblatt 85/22

(84) Benannte Vertragsstaaten:
AT BE CH DE FR GB IT LI LU NL SE

(56) Entgegenhaltungen:
CH - A - 518 104
US - A - 3 312 221
US - A - 3 473 532
US - A - 3 572 340
US - A - 4 014 337

(73) Patentinhaber: INTERMEDICAT GMBH,
Gerliswilstrasse 43, CH-6020 Emmenbrücke (CH)

(72) Erfinder: Leclerc, Roland-Yves Jacques, "La Groie",
F-28400 Nogent-le-Rotrou (FR)

(74) Vertreter: von Kreisler, Alek, Dipl.-Chem. et al,
Deichmannhaus am Hauptbahnhof, D-5000 Köln 1 (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Beschreibung

Die Erfindung betrifft eine Vorrichtung für eine medizinische Saugdrainage, bestehend aus einem evakuierbaren Behälter aus flexiblem Material in Form eines zusammenrollbaren Flachbehälters, Folienbeutels oder dgl., der mit sekretaufnehmendem Material gefüllt ist und mit einem sekretbildenden Körperhohlraum verbindbar ist, wobei in die Verbindung ein Rückschlagventil eingesetzt ist.

Es handelt sich um eine Vorrichtung und einen Behälter, der als Rezipient für abgesonderte Körperflüssigkeiten, z. B. Wundsekrete, dient und durch manuelle Behandlung als Unterdruckreservoir hergerichtet werden kann. Ab- oder adsorbierende Agentien im Innenraum des flexiblen beutelartigen Behälters unterstützen durch kapillar- oder sorptionsphysikalische Aktivität die bei dem jeweils gegebenen Unterdruck stattfindende Exsudatdrainage.

Bei der postoperativen Wunddrainage ist es üblich, zur Ableitung während der Wundheilung entstehender Flüssigkeitsabsonderungen Katheter in die z. B. durch Nähte oder Wundklammern umschlossenen Operationswunden gewebeschlüssig einzulegen, die nach erfolgter Heilung durch Herausziehen entfernt werden. Es hat sich als vorteilhaft erwiesen, auf die dabei verwendeten, über einen Teil ihrer Länge vielfach perforierten flexiblen Drainagekatheter einen Unterdruck wirken zu lassen, der bewirkt, daß erstens entstehendes Exsudat möglichst rasch entfernt wird und zweitens die Schnittflächen in der Operationswunde aneinandergepreßt gehalten werden und so eine rasche Gewebsgranulation und Wundheilung einsetzt. Systeme, die aus einem flexiblen, vielfach in der Katheterwand gelochten Drainageschlauch und einem angeschlossenen, vorevakuierten Unterdruckreservoir bestehen, sind z. B. als Redondrainagesysteme bekannt.

Diese Drainagesysteme sind in Ausführungen sowohl für den Mehrfach- als auch für den aus hygienischen und arbeitsökonomischen Gründen vorteilhaften Einmalgebrauch üblich und haben als Vakuumquelle z. B. eine auf ein bestimmtes Vorvakuum von z. B. 0,1 bar evakuierte Glasflasche. Das bestehende Vakuum kann in vielen Fällen durch eine Vakuumanzeige qualitativ abgelesen werden. Dieses nicht stationäre System kann von ambulanten Patienten getragen werden, ist aber wegen der Bruchanfälligkeit, des Gewichts und der geometrisch ungünstigen Form des Glasbehälters sicher nicht optimal, zumal die für die Formbeständigkeit unter hohem Vakuum erforderliche Schlauchwanddicke ein unhandliches und relativ starres Schlauchableitungssystem bedingt. Die üblicherweise aus Kunststoffen gefertigten, zum Einmalgebrauch vorgesehenen Geräte sind in dieser Hinsicht oft günstiger, da zumindest leicht und unzerbrechlich. Sie bestehen z. B. aus einem flexiblen, ziehharmonikaähnlich zusammendrückbaren und oft durch Federkraft auseinandergedrückten Kunststoffbehälter, in dem durch z. B. Zusammendrükken ein luftleerer Innenraum geschaffen wird und der nach Anschluß des Drainagekatheters durch eingebaute Expandierhilfen die notwendige Sogwirkung entfaltet.

Obwohl es wegen der besseren Wundheilung angezeigt ist, einen relativ hohen Unterdruck in der Vakuumquelle vorrätig zu haben, ist dies andererseits nachteilig, da bei hohem Vakuum das außen am Katheter in der Wunde anliegende Gewebe, insbesondere bei Gehirn- und Mammaoperationen, in die Löcher des Katheters gesaugt und gepreßt wird und das dann beim Entfernen, d. h. beim Herausziehen des Katheters, wieder aufgerissen wird und ein erneutes Trauma in der früheren Wunde herstellt. Da das Exsudat als im Behälter beweglich vorhandene Flüssigkeit vorliegt, ist auch hierin ein gewisser Nachteil zu sehen, da bei Unvorsichtigkeit das unter Umständen infizierte Exsudat wieder in den Katheter zurückströmen kann oder bei Verwendung von Glasflaschen bei Bruch der Flasche nach außen gelangt und die Umgebung und das Pflegepersonal kontaminieren kann.

Diesen Mängeln hilft eine bekannte Vorrichtung (US-A-3 572 340) ab, die aus einem evakuierbaren Beutel-Behälter aus flexiblem Material besteht, der mit Material gefüllt ist, das unter Volumenausdehnung Sekret aufnimmt. In der Mitte zweier gegenüberliegender Ränder des Behälters sind mit Stutzen versehene Öffnungen ausgebildet, die als Sekreteinlaß bzw. Luftauslaß dienen. Der Sekreteinlaßöffnung ist ein Kugelventil zugeordnet, das ein Rückströmen von Sekret in den Patienten verhindern soll. Das Sekret, das aus den Wunden abgesaugt wird, besteht aus einer kolloidhaltigen Proteinlösung, die den Nachteil hat, das betreffende Kugelventil durch Verkleben zu verstopfen, insbesondere da ja die Beutel bei Körpertemperaturen getragen werden und eine Gerinnung des Sekretes bei diesen Temperaturen besonders leicht eintritt. Wenn zur Erzielung ausreichender Entlüftung durch Zusammenrollen des Behälters über eine große Wickellänge nur ein Stutzen vorgesehen wird, ergeben sich Schwierigkeiten bei der Entlüftung, weil das Kugelventil außer Aktion gesetzt werden muß. Zu diesem Zweck ist es in einem separaten Teil untergebracht, der mit dem Einlaßstutzen nach der Evakuierung zusammengesteckt wird. Dieser Teil verteuert die Vorrichtung zur Einmalanwendung unangemessen. Auch die Ausbildung des Ventils als Kugelventil trägt hierzu bei.

Es stellt sich daher die Aufgabe, eine für die Wunddrainage geeignete billige Vorrichtung zum Einmalgebrauch zu schaffen, die sich schnell und einfach handhaben läßt, um das zur maximalen Ausnutzung der Saugwirkung des sekretaufnehmenden Materials erforderliche Vakuum manuell zu erzeugen.

Die Aufgabe wird durch die in den Ansprüchen gekennzeichnete Erfindung gelöst. Die Ausbildung des Ventils als Folienventil gestattet das Zusammenrollen des Behälters über einen verhältnismäßig langen Wickelweg, so daß eine sehr gute Entlüftung und eine maximale Ausnutzung der Saugwirkung des Füllmaterials erreicht werden. Außerdem verklebt dieses Ventil nicht durch den Einfluß gerinnenden Sekrets. Es ist vorgesehen, daß ein aus einer unter anderem geruchs- und bakteriendichten, transparenten Kunststoffolie im Schweißverfahren hergestellter beutelartiger Behälter mit einem offenporigen, in hohem Grade flüssigkeitsaufnehmenden schwammartigen Material gefüllt ist, das in der Lage ist, Wundsekret aufzunehmen und auch bei Anwesenheit von Wundsekret durch Eigenelastizität den komprimierten Behälter unter Erzeugung einer Sogwirkung aufrichten und ausdehnen kann. Flüssigkeitssorbierende und dabei mit Volumenvergrößerung reagierende Stoffe, die dabei die gleiche Wirkung entfalten, sind ebenfalls geeignete Füllmaterialien.

Als offenporiges Füllmaterial schwammartiger Struktur sind z. B. Schaumstoffe aus Polyurethan mit geeigneter Rückstellelastizität zu nennen, als Volumenvergrößerung reagierendes Sorptionsmaterial ist z. B. ein unter der Bezeichnung »Favor®« der Firma Stockhausen bekanntes Absorbens zu nennen. Selbstverständlich sind auch Kombinationen beider Materialien möglich. Bakterizid wirkende Mittel können in den Schaumstoff eingearbeitet werden, um bei evtl. Verkeimung ein Wachstum bzw. eine dabei entstehende Gasbildung auszuschließen.

Ein zwischen den beiden den Behälter bildenden Folien eingeschweißter Schlauch dient als Zuleitung in das Behälterinnere und bildet, wenn vollständig gefüllt, in Verbindung mit dem sorbierenden oder flüssigkeitsaufnehmenden Medium die für die kapillaraktive Wirkung notwendige Flüssigkeitszuführung.

Vorteilhafterweise ist vorgesehen, daß infolge des geringen Volumens Schläuche mit geringer Shore-Härte für die Drainageleitung verwendet werden, die eine größere Flexibilität bei der Anlage und sicheres Verweilen des Drains im Wundgebiet zur Folge haben. Die zur Einleitung der kapillaraktiven Wirkung erforderliche Flüssigkeitssäure kann durch Anlegen eines temporär wirkenden geringen Vakuums, z. B. durch Aufrollen des evakuierten, verschlossenen Behälters, gebildet und damit an das sorbierende Medium herangeführt werden. Der Schlauch kann am Ende einen für lösbare Verbindungen erforderlichen üblichen Ansatz aufweisen, an dem eine schraubbare oder steckbare, aus männlichem und weiblichem Kegelansatz bestehende Verbindung zum Auswechseln des Behälters hergestellt werden kann.

Zur Unterbrechung der die Drainage fördernden Sogwirkung ist an diesem schlauchartigen Beutelzugang eine Sperrvorrichtung vorgesehen, die im einfachsten Falle als sog. Schiebeklemme den Schlauch umfaßt und beim Verschieben durch die spitzwinklig geformte Aussparung das Schlauchlumen kontinuierlich eng. Eine andere Sperrmöglichkeit ist ein in den Schlauch eingefügtes Nadelventil, bestehend aus Gehäuse und Ventilspindel. Ein nach Bedarf in seiner Länge festzulegendes weiteres Schlauchstück führt von der lösbaren Verbindungsstelle weiter und endet in einem Schlauchansatz, der an den in der Wunde plazierten Drainagekatheter angeschlossen werden kann. Befestigungsösen an der Oberseite des Behälters erlauben es, daß er mit geeigneten Aufhänge- oder Befestigungsmitteln am ambulanten Patienten selbst oder aber an einer externen Vorrichtung festgemacht werden kann. Je nach Applikationsort kann der Behälter in anatomisch angepaßter Form und dem Sekretanfall entsprechender Größe ausgelegt werden.

Der zur behandlungsgerechten Wundsekretdrainage erforderliche Unterdruck bzw. die Sogwirkung wird mit und in dem erfindungsgemäßen Behälter erzeugt, indem in einfacher Weise das enthaltene Luftvolumen z. B. durch Falten oder Zusammenrollen des Behälters entfernt wird, der komprimierte Behälter dann vorübergehend mit der Sperrvorrichtung abgeschlossen und nach Verbindung mit dem Katheter der gesamte Drainageweg geöffnet wird. Sowohl Unterdruck als auch Kapillarwirkung bzw. Sorptionseigenschaften des im Behälter befindlichen Füllmaterials bewirken dann unter Behälterexpansion eine schonende Sekretabführung und Aufnahme im Behälter selbst. Gegebenenfalls kann das Vakuum auch durch Evakuieren mit einer externen Vakuumquelle erzeugt werden.

Der Zuleitungsschlauch mit dem Folienventil kann sich in der Mitte oder an der Seite eines Randes des Behälters befinden. Dementsprechend kann der beutelartige Behälter zu seiner Entlüftung von dem zum Zuleitungsschlauch quer gerichteten gegenüberliegenden Rand oder von dem zum Zuleitungsschlauch parallelen Rand her zusammengerollt werden, so daß der Wickelweg für den Behälter immer lang ist. Das Folienventil ist billig und es wird gemeinsam mit dem Zuleitungsschlauch, an dem es befestigt ist, mit dem Behälter verbunden. Zu diesem Zweck wird lediglich der Behälterrand, durch den der Zuleitungsschlauch hindurchragt, zugeschweißt. Das Folienventil wird zur Entlüftung des Behälters unwirksam gemacht, indem ein dünner Schlauch durch den Zuleitungsschlauch und das Folienventil hindurch in den Behälter eingeführt wird. Die Luft entweicht beim Zusammenrollen des Behälters durch den dünnen Schlauch und sobald er herausgezogen worden ist, schließt sich das Folienventil und tritt zur Verhinderung des Lufteintritts in den Behälter bzw. Rückströmung von Sekret aus dem Behälter in Aktion. Es gewährleistet die hygienische und zuverlässige Funktion der Vorrichtung.

Ausführungsbeispiele des erfindungsgemäßen Behälters sind in den Abbildungen dargestellt. Es zeigt

Fig. 1 eine schaubildliche Ansicht einer Ausführungsform der Erfindung;

Fig. 2 die Vorrichtung nach Fig. 1 in zusam-

mengerolltem Zustand;

Fig. 3 einen Schnitt längs der Linie III-III in Fig. 1 und

Fig. 7 einen Längsschnitt des geschlossenen Rückschlagventils.

Die Vorrichtung besteht im wesentlichen aus einem Behälter 12 in Form eines flachen Folienbeutels, dessen Hohlraum vollständig mit einem sorbierenden Material 16 gefüllt ist, das als dicke Werkstoffbahn gestaltet ist. Der Behälter 12 ist ringsum geschlossen und weist an einem Rand zwei Befestigungsösen 17 auf, mit deren Hilfe er am Patienten oder einer Haltevorrichtung aufgehängt werden kann.

In den Behälter 12 mündet ein Zuleitungsschlauch 11, der eine Hülse 15 einer Steckkupplung zum Anschluß an einen in eine Körperhöhle eingesetzten Katheter trägt. Der Zuleitungsschlauch 11 ist in einen Behälterrand eingeschweißt. Weitere Auslaß- oder Einlaßöffnungen sind an dem Behälter 12 nicht vorgesehen.

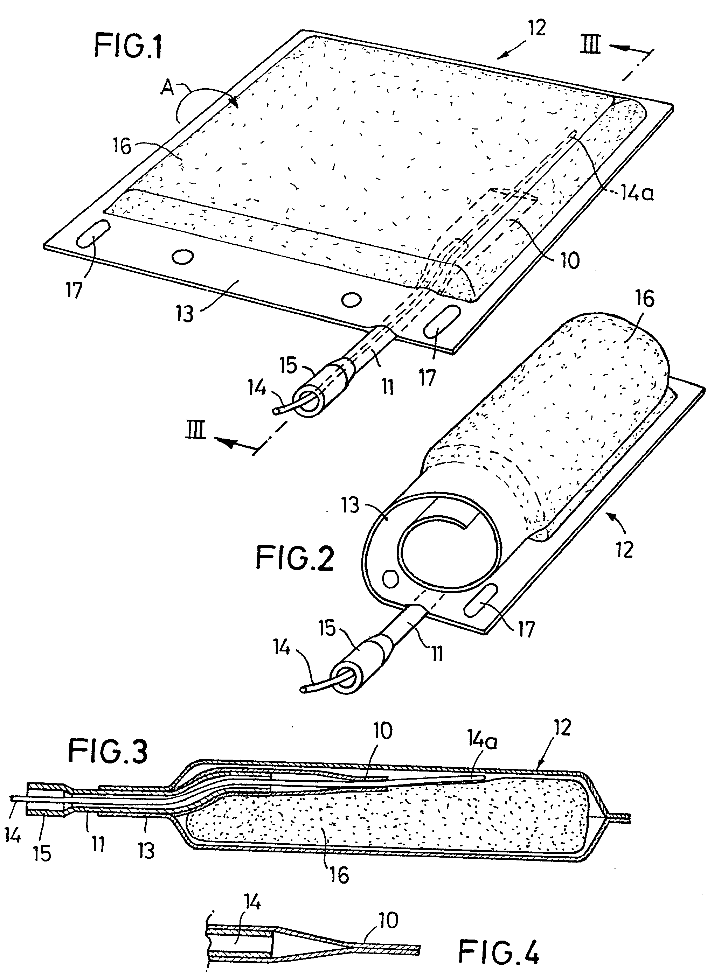

Durch Zusammenrollen des Behälters 12 (Fig. 2) wird das Luftvolumen aus dem Behälter 12 herausgedrückt. Unterdruck und Kapillarwirkung des sorbierenden Materials 16 bewirken unter Ausdehnung des Behälters 12 eine Sekretabziehung und Einführung in den Behälter 12.

Als Material 16 kann anstatt eines geeigneten Schaumstoffes Laminaria (Seetang) in körniger Form oder als Zusatz zu Schaumstoff-Formteilen, verwendet werden. Laminaria quillt durch Flüssigkeitsaufnahme und gibt die Flüssigkeit nicht wieder ab. Die Saugwirkung dieses Materials ist beträchtlich, und der Unterdruck wird nicht als Soghilfe benötigt.

Bei der gezeichneten Ausführungsform ist der Zuleitungsschlauch 11 an der Seite des Randes 13 des Behälters 12 angeordnet und mit dem Rand 13 verschweißt. In dem Rand 13 sind die Löcher 17 zur Befestigung des Behälters 12 am Körper des Patienten angeordnet. Am äußeren Ende des Zuleitungsschlauches 11 ist die Hülse 15 einer Steckkupplung zum Anschluß eines Drainageschlauches befestigt, während am inneren Ende des Zuleitungsschlauches 11 ein Flatterventil 10 angebracht ist. Das Flatterventil 10 besteht aus zwei aufeinanderliegenden Folien, die an ihren Längsrändern miteinander verbunden und unten offen belassen sind. Der Behälter 12 wird in Richtung des Pfeiles A parallel zum Zuleitungsschlauch 11 zusammengerollt. Um dabei das Flatterventil 10 auszuschalten, wird durch die Hülse 15, den Zuleitungsschlauch 11 und das Flatterventil 10 ein dünner Schlauch 14 geschoben, dessen inneres Ende 14a aus dem Flatterventil 10 heraus in den Behälter 12 hineinragt und die Luft aus dem Behälter 12 herausführt. Nach ausreichender Entlüftung des Behälters 12 wird der dünne Schlauch 14 herausgezogen, das Flatterventil 10 schließt sich und der Unterdruck in dem Behälter 12 kann zum Absaugen von Sekret aus einer Körperhöhle in sorbierendes Material 16 im Inneren des Behälters 12 aus Folienmaterial ausgenutzt werden. Die Saugwirkung des Unterdruckes wird von der Kapillarwirkung des sekretaufnehmenden Materials 16 unterstützt.

**Patentansprüche**

1. Vorrichtung für eine medizinische Saugdrainage, bestehend aus einem evakuierbaren Behälter (12) aus flexiblem Material in Form eines zusammenrollbaren Flachbehälters, Folienbeutels oder dgl., der mit sekretaufnehmendem Material (16) gefüllt ist und mit einem sekretbildenden Körperhohlraum verbindbar ist, wobei in die Verbindung ein Rückschlagventil (10) eingesetzt ist, dadurch gekennzeichnet, daß das Rückschlagventil als Folienventil (Flatterventil 10) ausgebildet und innerhalb des Behälters (12) angeordnet ist.

2. Vorrichtung nach Anspruch 1, dadurch gekennzeichnet, daß das sekretaufnehmende Material (16) ein rückstellfähiges, komprimierbares offenporiges Material ist.

3. Vorrichtung nach Anspruch 1, dadurch gekennzeichnet, daß das sekretaufnehmende Material (16) ein offenporiges, in trockenem Zustand starres Medium ist, das bei Sekretaufnahme unter Volumenausdehnung weich-flexibel wird.

4. Vorrichtung nach Anspruch 1, dadurch gekennzeichnet, daß das sekretaufnehmende Material (16) ein Werkstoff ist, welcher durch molekulare Bindekräfte Sekretflüssigkeit unter Volumenausdehnung aufnimmt.

5. Vorrichtung nach einem der Ansprüche 1—4, dadurch gekennzeichnet, daß sowohl ein offenporiges und starres Medium als auch ein infolge molekularer Bindekräfte sekretaufnehmendes Medium im Behälter (112) vorhanden sind.

6. Vorrichtung nach einem der Ansprüche 1—5, dadurch gekennzeichnet, daß bakterizid wirkende Mittel in das sekretaufnehmende Material (16) eingearbeitet sind.

7. Vorrichtung nach einem der Ansprüche 1—6, dadurch gekennzeichnet, daß das sekretaufnehmende Material (16) aus Laminaria (Seetang) in körniger Form oder aus in Formteile in den offenporigen Schaum eingearbeiteter Laminaria besteht.

8. Vorrichtung nach einem der Ansprüche 1—7, dadurch gekennzeichnet, daß das Behältermaterial aus weitgehend gasundurchlässiger polymerer Mono- oder Verbundfolie besteht.

9. Vorrichtung nach einem der Ansprüche 1—7, dadurch gekennzeichnet, daß das Behältermaterial aus einer metallisierten polymeren Mono- oder Verbundfolie besteht.

10. Vorrichtung nach einem der Ansprüche 1—9, dadurch gekennzeichnet, daß sie zur Saugdrainage sterilisierbar ist.

11. Vorrichtung nach einem der Ansprüche 1—10, dadurch gekennzeichnet, daß der Zuleitungsschlauch aus einem Schlauchmaterial geringerer Shore-Härte besteht.

12. Vorrichtung nach einem der Ansprüche 1—11, dadurch gekennzeichnet, daß am Zuleitungsschlauch eine kontinuierlich wirkende Absperrvorrichtung angeordnet ist.

13. Vorrichtung nach einem der Ansprüche 1 – 12, dadurch gekennzeichnet, daß der Zuleitungsschlauch in der Mitte eines Randes des Behälters angeordnet ist.

14. Vorrichtung nach einem der Ansprüche 1 – 12, dadurch gekennzeichnet, daß der Zuleitungsschlauch (11) an einer Seite eines Randes (13) des Behälters (12) angeordnet ist.

15. Vorrichtung nach Anspruch 1, dadurch gekennzeichnet, daß zur Öffnung des Folienventils (Flatterventil 10) ein dünner Schlauch (14) vorgesehen ist, der länger ist als der Zuleitungsschlauch (11) mit dem Folienventil (Flatterventil 10) und der aus dem Folienventil (Flatterventil 10) und dem Zuleitungsschlauch (11) herausziehbar ist.

16. Vorrichtung nach Anspruch 1, dadurch gekennzeichnet, daß der Behälter (12) rechteckig gestaltet ist und daß sich der Zuleitungsschlauch (11) an der einen Längsseite des Behälters (12) befindet.

## Claims

1. A medical suction drainage device consisting of an evacuatable receptacle (12) of a flexible material in the form of a flat receptacle, film receptacle or the like capable of being rolled, which has been filled with a material (16) sorbing a secreted fluid and is adapted to be communicated to a secretionforming body cavity, a return check valve (10) having been inserted in the communication, characterized in that the return check valve has been formed as a film valve (flutter valve 10) and disposed within the receptacle (12).

2. The device according to claim 1, characterized in that the fluid-sorbing material (16) is a resilient compressible open-pore material.

3. The device according to claim 1, characterized in that the fluid-sorbing material (16) is an open-pore medium which is rigid in the dry state and which becomes soft-flexible and expands in volume upon sorbing the fluid.

4. The device according to claim 1, characterized in that the fluid-sorbing material (16) is a material which sorbs secreted fluid by molecular cohesion with a concomitant expansion in volume.

5. The device according to any one of claims 1 to 4, characterized in that there are present in the receptacle (12) an open-pore and rigid medium as well as a medium which sorbs secreted fluid by molecular cohesion.

6. The device according to any one of claims 1 to 5, characterized in that bactericidal agents have been incorporated in the fluidsorbing material (16).

7. The device according to any one of claims 1 to 6, characterized in that the fluidsorbing material (16) consists of Laminaria (kelp) in granular form or of Laminaria having been incorporated as particles in the open-pore foam.

8. The device according to any one of claims 1 to 7, characterized in that the receptacle material consists of a mostly gasimpermeable polymeric mono-film or laminate film.

9. The device according to any one of claims 1 to 7, characterized in that the receptacle material consists of a metallized polymeric mono-film or laminate film.

10. The device according to any one of claims 1 to 9, characterized in that it is sterilizable for the suction drainage.

11. The device according to any one of claims 1 to 10, characterized in that the inlet tube consists of a material having a low Shore hardness.

12. The device according to any one of claims 1 to 11, characterized in that a continuously operative shutoff device is disposed at the inlet tube.

13. The device according to any one of claims 1 to 12, characterized in that the inlet tube has been arranged in the center of one edge of the receptacle.

14. The device according to any one of claims 1 to 12, characterized in that the inlet tube (11) has been arranged on a side of one edge (13) of the receptacle (12).

15. The device according to claim 1, characterized in that a thin tube (14) has been provided for opening the film valve (flutter valve 10) which tube is longer than the inlet tube (11) with the film valve (flutter valve 10) and which is adapted to be pulled out from the film valve (flutter valve 10) and the inlet tube (11).

16. The device according to claim 1, characterized in that the receptacle (12) has been made of rectangular shape and that the inlet tube (11) is present on one longitudinal side of the receptacle (12).

## Revendications

1. Dispositif de drainage par aspiration à usage médical, comportant un récipient (12) en matière souple, dans lequel on peut produire une dépression, ce récipient (12), de forme plate et adapté à s'enrouler sur lui-même, comme par exemple un sachet confectionné dans une matière en feuille mince, égant garni d'une substance absorbante (16) adaptée à recueillir les sécrétions d'une cavité du corps d'un patient, à laquelle le récipient (12) peut être raccordé par l'intermédiaire d'une soupape anti-retour (10); caractérisé en ce que la soupape anti-retour est constituée par une soupape à lèvres minces et souples (10), qui est disposée à l'intérieur du récipient (12).

2. Dispositif selon la revendication 1, caractérisé en ce que la substance (16) prévue pour absorber les sécrétions du corps du patient, est compressible, pourvue de pores ouverts, et capable de reprendre élastiquement sa forme primitive.

3. Dispositif selon la revendication 1, caractérisé en ce que la substance (16) prévue pour absorber les sécrétions du corps du patient, est pourvue de pores ouverts, et constitue une matière rigide à l'état sec, cette substance augmentant de volume et devenant molle et souple lorsqu'elle absorbe les sécrétions.

4. Dispositif selon la revendication 1, caractérisé en ce que la substance (16) prévue pour absorber les sécrétions du corps du patient, est une matière qui augmente de volume en absorbant les sécrétions liquides, sous l'effet des forces de liaison moléculaires.

5. Dispositif selon l'une quelconque des revendications 1 à 4, caractérisé en ce que le récipient (12) contient à la fois une matière rigide à pores ouverts, et une matière capable d'absorber les sécrétions du corps du patient sous l'effet des forces de liaison moléculaires.

6. Dispositif selon l'une quelconque des revendications 1 à 5, caractérisé en ce que la matière (16) prévue pour absorber les sécrétions du corps du patient, contient des agents bactéricides.

7. Dispositif selon l'une quelconque des revendications 1 à 6, caractérisé en ce que la matière (16) prévue pour absorber les sécrétions du corps du patient, est constituée d'algues laminaires (varech) broyées en fragments, ou d'algues laminaires incorporées dans des pièces moulées en mousse à pores ouverts.

8. Dispositif selon l'une quelconque des revendications 1 à 7, caractérisé en ce que le récipient est réalisé en une matière polymère en feuille, sensiblement imperméable aux gaz, en une ou plusieurs épaisseurs composites.

9. Dispositif selon l'une quelconque des revendications 1 à 7, caractérisé en ce que le récipient est réalise en une matière polymère en feuille métallisée, en une ou plusieurs épaisseurs composites.

10. Dispositif selon l'une quelconque des revendications 1 à 9, caractérisé en ce que qu'il est stérilisable, en vue d'assurer un drainage par aspiration.

11. Dispositif selon l'une quelconque des revendications 1 à 10, caractérisé en ce que le tuyau d'arivée est constitué d'une matière de faible dureté Shore.

12. Dispositif selon l'une quelconque des revendications 1 à 11, caractérisé en ce qu'un organe d'arrêt à effet continu est monté sur le toyau d'arrivée.

13. Dispositif selon l'une quelconque des revendications 1 à 12, caractérisé en ce que le tuyau d'arrivée est disposé au milieu d'une bordure du récipient.

14. Dispositif selon l'une quelconque des revendications 1 à 12, caractérisé en ce que le tuyau d'arrivée (11) est disposé d'un côté d'une bordure (13) du récipient (12).

15. Dispositif selon la revendication 1, caractérisé en ce que le tuyau de petit calibre (14) est prévu pour ouvrir la soupape anti-retour (10) à lèvres souples, ce tuyau d'ouverture (14) ayant une longueur supérieure à celle du tuyau d'arrivée (11) prolongé par la soupape anti-retour (10), ce tuyau d'ouverture (14) étant disposé de manière à être ensuite retiré hors de la soupape anti-retour (10), et hors du tuyau d'arrivée (11).

16. Dispositif selon la revendication 1, caractérisé en ce que le récipient (12) est de forme rectangulaire, et en ce que le tuyau d'arrivée (11) se trouve à l'endroit de l'un des grands côtés du récipient (12).

FIG.1

A

16

12

III

14a

17

13

10

15

14

11

17

III

FIG.2

16

13

12

15

14

11

17

FIG.3

10

14a

12

14

15

11

13

16

14

10

FIG.4